# EUROPÄISCHE PATENTSCHRIFT

(11) **EP 1 476 084 B1**
(45) Veröffentlichungstag und Bekanntmachung des Hinweises auf die Patenterteilung: **14.06.2006**
(21) Anmeldenummer: 03704476.5
(22) Anmeldetag: 28.01.2003
(51) Int. Cl.: A61B 17/15

(54) **SÄGELEHRE FÜR MEDIZINISCHE ZWECKE**
SAW JIG FOR MEDICAL PURPOSES
GABARIT DE SCIAGE POUR APPLICATIONS MEDICALES

(30) Priorität: 13.02.2002 DE 10205799
(43) Veröffentlichungstag der Anmeldung: 17.11.2004
(73) Patentinhaber: Karl Storz GmbH & Co. KG, 78532 Tuttlingen (DE)
(72) Erfinder: EGGER, Berthold, 78333 Stockach (DE); BECK, Rüdiger, 78333 Stockach (DE)
(74) Vertreter: Hofmeister, Frank
(86) Internationale Anmeldenummer: PCT/EP2003/000813
(87) Internationale Veröffentlichungsnummer: WO 2003/068075

(56) Entgegenhaltungen:
- DE-A- 19 516 294
- US-A- 4 502 474
- US-A- 4 750 481
- US-A- 5 601 565

## Beschreibung

Die Erfindung betrifft eine Sägelehre für medizinische Zwecke, mit einer Handhabe und einem an der Handhabe gelagerten Sägeblattführungselement zur führenden Aufnahme eines Sägeblattes, wobei das aus einem an der Handhabe gelagerten Rahmen und einer in dem Rahmen verschwenkbar gelagerten Führungsaufnahme für das Sägeblatt bestehende Sägeblattführungselement auf dem Sägegut fixierbar ist und in der auf dem Sägegut fixierten Stellung auf unterschiedliche Sägeschnitte einstellbar ist.

Sägelehren werden verwendet, um bei einem Sägevorgang das Sägeblatt der Säge in einer exakt ausgerichteten Winkellage zum zu sägenden Sägegut zu führen. Eine solche exakte Führung des Sägeblattes ist insbesondere bei Sägelehren für medizinische Zwecke erforderlich, wenn, wie beispielsweise bei Hallux valgus Operationsverfahren zur Beseitigung von Belastungsdeformationen im Bereich der Großzehe, Knochen mittels einer Säge bearbeitet werden müssen.

Eine gattungsgemäße Sägelehre für medizinische Zwecke ist beispielsweise aus der US 5 911 724 A bekannt. Bei dieser bekannten Sägelehre besteht das Sägeblattführungselement aus einem am Sägegut festlegbaren Rahmen, einem mit dem rahmen verbundenen Winkelmesser sowie einer über einen Schwenkarm am Winkelmesser verstellbar festlegbaren Sägeblattführung. Zwar ermöglicht diese bekannte Sägelehre das Einstellen verschiedener Sägeschnitte, während die Sägelehre am Sägegut fixiert ist, jedoch besteht diese bekannte Vorrichtung aus sehr vielen separat einzustellenden Bauteilen, die einerseits die Fixierung und Einstellung verkomplizieren und andererseits durch ihr wechselseitiges Spiel eine exakte Führung des Sägeblatts erschweren.

Eine weitere Sägelehre ist aus der DE-A-195 16 294 bekannt. Bei dieser bekannten Vorrichtung besteht die Sägeblattführung aus einer Führungsschiene, die über ein Verbindungsstück an einem Kugelgelenk gelagert ist, das seinerseits verschwenkbar zwischen zwei Spannbacken angeordnet ist. Zwar ermöglicht die Lagerung der Führungsschiene an dem Kugelgelenk einen großen Verschwenkwinkel der zur Aufnahme des Sägeblattes dienenden Führungsschiene, jedoch ist die exakte Einstellung einer bestimmten Winkelposition kompliziert einzustellen, da der Operateur mit einer Hand die Führungsschiene in der exakten Position halten muss, während er mit der anderen Hand die Spannbacken zum Fixieren des Kugelgelenks gegeneinander über eine Schraubverbindung verspannt.

Aus der Praxis der Hallux valgus Operationsverfahren ist eine weitere Gabel-Sägelehre für medizinische Zwecke bekannt, die mittels eines Drahtes auf dem zu bearbeitenden Knochen fixierbar ist. Zwar ermöglicht die gabelförmige Sägeblattführung eine Ausrichtung des Sägeblattes, jedoch muss der Operateur die Winkellagen selbst bestimmen und halten, wodurch eine präzise Ausbildung eines beispielsweise keilförmigen Sägeschnittverlaufs kaum praktikabel ist.

Davon ausgehend liegt der Erfindung die **Aufgabe** zugrunde, eine Sägelehre für medizinische Zwecke zu schaffen, die einfach zu handhaben ist und eine exakte und positionsgenaue Führung des Sägeblattes gewährleistet.

Die **Lösung** dieser Aufgabenstellung ist erfindungsgemäß dadurch gekennzeichnet, dass der Rahmen als einseitig offener Kreisringabschnitt ausgebildet ist, in den die einen im wesentlichen kreisförmig ausgebildeten Grundkörper aufweisende Führungsaufnahme formschlüssig einsetzbar ist, an deren Grundkörper ein sich radial nach außen erstreckender Steg angeformt ist und zumindest im Steg ein im wesentlichen senkrecht zur horizontalen Erstreckung des Sägeblattführungselements verlaufender Schlitz zur Aufnahme des Sägeblattes ausgebildet ist, und wobei der Verschwenkwinkel der Führungsaufnahme innerhalb des Rahmens durch die Anlage des Stegs an den beiderseitigen Stirnflächen der Öffnung des Rahmens begrenzt ist.

Durch die erfindungsgemäße Ausgestaltung wird dem Operateur erstmalig eine Sägelehre an die Hand geliefert, die eine stets exakte und positionsgenaue Führung des Sägeblattes gewährleistet und die Einstellung verschiedener Schnittstellungen bei Beibehaltung der Position des auf dem Sägegut fixierten Rahmens ermöglicht.

Der in dem Steg ausgebildete Schlitz zur Aufnahme des Sägeblattes erstreckt sich vorteilhafterweise bis in die Mitte des Grundkörpers.

Um auch das Einführen eines Sägeblattes mit geschränkten Sägezähnen in den führenden Schlitz der Führungsaufnahme zu ermöglichen, ohne daß sich die Sägezähne in der Sägelehre verkeilen, ist der Schlitz im Bereich der Sägezähne des Sägeblattes breiter ausgestaltet.

Der Öffnungswinkel des in der Form eines offenen Kreisringabschnitts ausgebildeten Rahmens ist kleiner 180°, um eine führende Lagerung der Führungsaufnahme im Rahmen sicherzustellen, bei der der Rahmen die Führungsaufnahme um mehr als die Hälfte ihrer Umfangsfläche umschließt. Vorzugsweise beträgt der Offnungswinkel 60°.

Um die Führungsaufnahme im Rahmen sicher führen und positionieren zu können, sind am Rahmen mindestens ein Führungselement und mindestens ein Arretierungsselement angeordnet. Gemäß einer praktischen Ausführungsform ist dabei in der radial nach außen weisenden Umfangsfläche des Grundkörpers mindestens eine Nut zur Aufnahme jeweils eines am Rahmen gelagerten Führungselements ausgebildet, wobei am Rahmen vorteilhafterweise zwei sich im wesentlich einander gegenüberliegend angeordnete Führungselemente gelagert sind und das mindestens eine Führungselement als derart in den Rahmen einschraubbare Schraube ausgebildet ist, dass das den Rahmen durchragende freie Ende der Schraube führend in die Nut des Grundkörpers eingreift.

Zum lagegenauen Positionieren der Führungsaufnahme innerhalb des Rahmens ist die Führungsaufnahme über ein als Stellschraube ausgebildetes Arretierungsselement in der jeweiligen Verschwenkposition innerhalb des Rahmens festlegbar.

Der Verschwenkwinkel der Führungsaufnahme innerhalb des Rahmens ist durch die Anlage des Stegs an den beiderseitigen Stirnflächen der Öffnung des Rahmens begrenzt. Vorteilhafterweise wird deshalb der Öffnungswinkel des Rahmens so gewählt, dass er dem auszuführenden Schnittwinkel entspricht, da durch die drehbare in Anschlagendstellungen einzustellenden Schnittpositionen dem Operateur auf einfache Art und Weise das Durchführen eines immer exakten Sägeschnitts ermöglicht wird.

Das Fixieren der Sägelehre am Sägegut erfolgt über mindestens ein Fixierungselement, das gemäß einer ersten Ausführungsform der Erfindung als am Rahmen und/oder an der Führungsaufnahme, insbesondere am Grundkörper, ausgebildete Durchgangsbohrung ausgebildet ist, in die ein bis in das Sägegut reichender Haltestift, insbesondere ein Draht, einsetzbar ist. Vorteilhafterweise ist die Sägelehre über mindestens zwei Fixierungselement am Sägegut festlegbar, um ein Verdrehen der Sägelehre zu verhindern.

Gemäß einer zweiten Ausführungsform der Erfindung wird vorgeschlagen, dass das Fixierungselement als am Rahmen und/oder an der Führungsaufnahme, insbesondere am Grundkörper, ausgebildeter, am Sägegut festlegbarer Fixierungsdorn ausgebildet ist.

Das Einstellen neuer Schnittwinkel wird dadurch erleichtert, dass der Rahmen auswechselbar an der Handhabe gelagert ist, so dass durch einfaches Austauschen des Rahmens die Sägelehre mit neuen Anschlagwinkeln ausrüstbar ist.

Weiterhin wird mit der Erfindung vorgeschlagen, dass die Handhabung der erfindungsgemäßen Sägelehre dadurch erleichtert wird, dass die Handhabe als gegenüber dem Sägeblattführungselement abgewinkelter stielförmiger Griff ausgebildet ist, der vorteilhafterweise sowohl in horizontaler Richtung als auch in vertikaler Richtung gegenüber dem Sägeblattführungselement abgewinkelt ist.

Schließlich wird mit der Erfindung vorgeschlagen, dass die Oberfläche der Handhabe zumindest des distalen Teils der Handhabe gerändelt ausgebildet ist, um die Griffigkeit der Handhabe für den Operateur zu verbessern.

Weitere Merkmale und Vorteile der Erfindung ergeben sich anhand der zugehörigen Zeichnung, in der ein Ausführungsbeispiel einer erfindungsgemäßen Sägelehre nur beispielhaft schematisch dargestellt ist. In der Zeichnung zeigt:
- Fig. 1: eine Draufsicht auf eine erfindungsgemäße Sägelehre;
- Fig. 2: eine um 90° gedrehte Seitenansicht der Sägelehre gemäß Fig. 1;
- Fig. 3: einen Schnitt entlang der Schnittlinie III-III gemäß Fig. 1 und
- Fig. 4: eine teilweise geschnittene, ausschnittweise schematische Seitenansicht einer erfindungsgemäßen Sägelehre mit eingesetztem Sägeblatt.

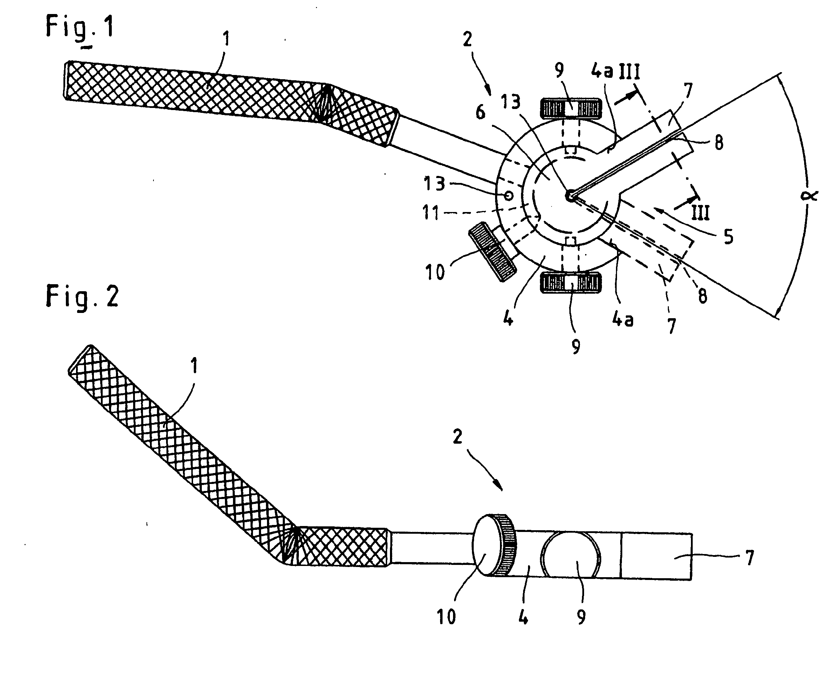

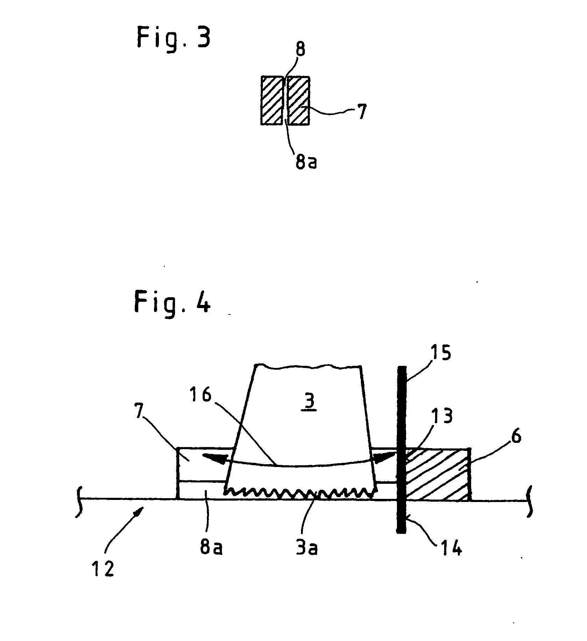

Die in den Abbildungen Fig. 1 und 2 dargestellte Sägelehre besteht im wesentlichen aus einer Handhabe 1 zum Führen und Halten der Sägelehre sowie einem an der Handhabe 1 gelagerten Sägeblattführungselement 2 zur führenden Aufnahme eines Sägeblattes 3, wie dies in Fig. 4 dargestellt ist. Zur Verbesserung der Handhabbarkeit ist die Handhabe 1, wie aus den Abbildungen ersichtlich, gegenüber dem Sägeblattführungselement 2 abgewinkelt, beim dargestellten Ausführungsbeispiel sogar zweimal, nämlich in horizontaler und vertikaler Richtung bezüglich der Erstreckung des Sägeblattführungselements 2. Zusätzlich ist die Oberfläche der Handhabe 1 zumindest im distalen Griffbereich gerändelt ausgebildet, um die Griffigkeit des stielförmigen Griffs zu erhöhen.

Das Sägeblattführungselement 2 besteht bei der dargestellten Ausführungsform aus einem einseitig offenen, kreisringabschnittförmigen Rahmen 4 sowie einer gegenüber dem Rahmen 4 verschwenkbaren, formschlüssig in den Rahmen 4 eingesetzten Führungsaufnahme 5, die von einem im wesentlichen kreisförmigen Grundkörper 6 sowie einem sich vom Grundkörper 6 radial nach außen erstrekkenden Steg 7 gebildet wird. Wie aus Fig. 1 ersichtlich, beträgt der Öffnungswinkel α des Rahmens 4 weniger als 180°, damit der Rahmen 4 den Grundkörper 6 der Führungsaufnahme 5 zumindest soweit umschließt, dass die Führungsaufnahme 5 innerhalb des Rahmens 4 gehalten wird und nur in vertikaler Richtung aus dem Rahmen 4 entnommen bzw. in diesen eingesetzt werden kann. Bei der dargestellten Ausführungsform des Rahmens 4 beträgt der Öffnungswinkel α 60°.

Zur Aufnahme und Führung des Sägeblattes 3 ist in der Führungsaufnahme 5 ein Schlitz 8 ausgebildet, der sich vom freien Ende des Stegs 7 bis zur Mitte des Grundkörpers 6 erstreckt. Um auch ein Sägeblatt 3 mit geschränkt ausgebildeten, das heißt seitlich leicht nach außen stehenden Sägezähnen 3a in den Schlitz 8 einsetzen und in diesem Führen zu können, ist der Schlitz 8 im unteren Bereich 8a der Sägezähne 3a breiter ausgebildet als im oberen Bereich, wie dies der Schnittdarstellung gemäß Fig. 3 zu entnehmen ist.

Das Führen und lagegenaue Positionieren der Führungsaufnahme 5 innerhalb des Rahmens 4 erfolgt über mindestens ein am Rahmen 4 angeordnetes Führungselement 9 sowie mindestens ein am Rahmen 4 angeordnetes Arretierungselement 10. Bei der dargestellten Ausführungsform der Sägelehre sind als Führungselemente 9 zwei sich am Rahmen 4 einander gegenüberliegende, in den Rahmen 4 einschraubbare Schrauben vorgesehen, deren den Rahmen 4 durchragenden freien Enden in eine in der radialen Umfangsfläche des Grundkörpers 6 ausgebildete Nut 11 so eingreifen, dass die Führungsaufnahme 5 einerseits in vertikaler Richtung sicher innerhalb des Rahmens 4 gehalten wird, andererseits aber weiterhin innerhalb des Rahmens 4 verschwenkbar gelagert ist.

Während die Führungselemente 9 ausschließlich dazu dienen, die Führungsaufnahme 5 verschwenkbar innerhalb des Rahmens 4 zu lagern, erfolgt das positionsgenaue unverrückbare Festlegen der Führungsaufnahme 5 über das als Stellschraube ausgebildete Arretierungselement 10, über welches die Führungsaufnahme 5 in ihrer jeweiligen Verschwenkposition innerhalb des Rahmens 4 festklemmbar ist. Der Verschwenkwinkel der Führungsaufnahme 5 innerhalb des Rahmens 4 wird durch die Anlage des Stegs 7 an den Stirnflächen 4a des Rahmens 4 begrenzt, wie dies der Abbildung gemäß Fig. 1 zu entnehmen ist, in der beide Endpositionen des Verschwenkbereichs der Führungsaufnahme 5 dargestellt sind. Vorteilhafterweise wird der Öffnungswinkel α des Rahmens 4 so gewählt, dass er dem vom Operateur auszuführenden keilförmigen Sägeschnitten entspricht, so dass der Operateur zur Einstellung der richtigen Schnittpositionen die Führungsaufnahme 5 jeweils nur bis zur Anlage an einer der Stirnflächen 4a des Rahmens 4 verschwenken muß, um die positionsgenaue Lage zur Ausführung des Sägeschnitts einzustellen.

### Die dargestellte Sägelehre wird wie folgt verwendet:

Vor der Operation bestimmt der Operateur die Größe des zu resezierenden Keils des Sägeguts und befestigt einen Rahmen 4 mit einem entsprechenden Öffnungswinkel α an der Handhabe 1. Dann positioniert der Operateur die Sägelehre mittels der Handhabe 1 auf dem Sägegut, einem Knochen 12, wie dies die Abbildung Fig. 4 zeigt, und fixiert die Sägelehre lagegenau am zu operierenden Knochen 12, wobei sich die Führungsaufnahme 5 in einer Anschlagendstellung befindet, in der der Steg 7 an einer Stirnfläche 4a des Rahmens 4 anliegt.

Zum Fixieren der Sägelehre am Sägegut (Knochen 12) weist die Sägelehre gemäß der dargestellten Ausführungsform zwei Durchgangsbohrungen 13 auf, die einerseits im Rahmen 4 und andererseits in der Mitte des Grundkörpers 6 der Führungsaufnahme 5 ausgebildet sind. Durch diese Durchgangsbohrungen 13 werden bis in entsprechende Bohrungen 14 im Knochen 12 reichende Haltestifte, beispielsweise Drähte 15, eingesetzt, über die die Sägelehre positionsgenau am Knochen 12 fixierbar ist, wie dies die Abbildung Fig. 4 am Beispiel der Durchgangsbohrung 13 in der Mitte des Grundkörpers 6 der Führungsaufnahme 5 zeigt.

Selbstverständlich besteht auch die Möglichkeit, an der Unterseite des Rahmens 4 und/oder der Führungsaufnahme 5 beispielsweise Fixierungsdorne anzuordnen, die in das Sägegut eindrückbar sind.

Nach dem Fixieren der Sägelehre am Knochen 12 führt der Operateur das Sägeblatt 3 vom freien Ende des Stegs 7 in den Schlitz 8 so ein, dass die vorteilhafterweise geschränkt ausgebildeten Sägezähne 3a im unteren breiteren Bereich 8a des Schlitzes 8 gelagert sind, wie dies ebenfalls der Abbildung Fig. 4 zu entnehmen ist. Das Sägeblatt 3 wird so weit in den Schlitz 8 eingeführt, bis es am Draht 15 anliegt. Nun kann der Operateur durch Ausführen einer Sägebewegung in Richtung des Pfeils 16 den ersten Sägeschnitt des zu resezierenden Keils ausführen. Anschließend zieht der Operateur das Sägeblatt 3 wieder über das freie Ende des Stegs 7 aus dem Schlitz 8 heraus.

Durch Öffnen des als Stellschraube ausgebildeten Arretierungselements 10 kann die Führungsaufnahme jetzt in die in Fig. 1 gestrichelt dargestellte zweite Anschlagendstellung verschwenkt werden, in der der Steg 7 an der anderen Stirnfläche 4a des Rahmens 4 anliegt. Nun wird das Sägeblatt 3 wieder in den Schlitz 8 eingeführt und der zweite, der den zu resezierenden Keil vollendende, Sägeschnitt ausgeführt.

Nach dem Herausziehen des Sägeblatts 3 aus der Führungsaufnahme 5 und dem Lösen der Sägelehre vom Knochen 12 erhält der Operateur einen exakt gesägten Keil, dessen Spreizwinkel genau dem Öffnungswinkel α des Rahmens 4 entspricht.

Eine solchermaßen ausgestaltete Sägelehre ermöglicht auf einfache und sichere Art und Weise eine stets exakte Führung und Positionierung des Sägeblatts 3, so dass der Operateur bei einfacher Handhabung einen immer exakten Sägeschnitt ausführen kann.

Darüber hinaus zeichnet sich die beschriebene Sägelehre dadurch aus, dass sie zu Reinigungszwecken einfach und schnell vollständig zerlegbar ist.

### Bezugszeichenliste

- 1: Handhabe
- 2: Sägeblattführungselement
- 3: Sägeblatt
- 3a: Sägezahn
- 4: Rahmen
- 4a: Stirnfläche
- 5: Führungsaufnahme
- 6: Grundkörper
- 7: Steg
- 8: Schlitz
- 8a: unterer Bereich
- 9: Führungselement
- 10: Arretierungselement
- 11: Nut
- 12: Knochen
- 13: Durchgangsbohrung
- 14: Bohrung
- 15: Draht
- 16: Pfeil (Sägebewegung)

- α: Öffnungswinkel

## Patentansprüche

1. Sägelehre für medizinische Zwecke, mit einer Handhabe (1) und einem an der Handhabe (1) gelagerten Sägeblattführungselement (2) zur führenden Aufnahme eines Sägeblattes (3), wobei das aus einem an der Handhabe (1) gelagerten Rahmen (4) und einer in dem Rahmen (4) verschwenkbar gelagerten Führungsaufnahme (5) für das Sägeblatt (3) bestehende Sägeblattführungselement (2) auf dem Sägegut fixierbar ist und in der auf dem Sägegut fixierten Stellung auf unterschiedliche Sägeschnitte einstellbar ist,
**dadurch gekennzeichnet,**
**dass** der Rahmen (4) als einseitig offener Kreisringabschnitt ausgebildet ist, in den die einen im wesentlichen kreisförmig ausgebildeten Grundkörper (6) aufweisende Führungsaufnahme (5) formschlüssig einsetzbar ist, an deren Grundkörper (6) ein sich radial nach außen erstreckender Steg (7) angeformt ist und zumindest im Steg (7) ein im wesentlichen senkrecht zur horizontalen Erstreckung des Sägeblattführungselements (2) verlaufender Schlitz (8) zur Aufnahme des Sägeblattes (3) ausgebildet ist, und wobei der Verschwenkwinkel der Führungsaufnahme (5) innerhalb des Rahmens (4) durch die Anlage des Stegs (7) an den beiderseitigen Stirnflächen (4a) der Öffnung des Rahmens (4) begrenzt ist.

2. Sägelehre nach Anspruch 1, **dadurch gekennzeichnet, dass** sich der Schlitz (8) bis in den Grundkörper (6), vorzugsweise bis in die Mitte des Grundkörpers (6), erstreckt.

3. Sägelehre nach Anspruch 1 oder 2, **dadurch gekennzeichnet, dass** der Schlitz (8) zur Aufnahme des Sägeblattes (3) im Bereich (8a) der Sägezähne (3a) des Sägeblattes (3) breiter ausgestaltet ist.

4. Sägelehre nach mindestens einem der Ansprüche 1 bis 3, **dadurch gekennzeichnet, dass** der Rahmen (4) einen Öffnungswinkel (α) von kleiner 180°, vorzugsweise 60°, aufweist.

5. Sägelehre nach mindestens einem der Ansprüche 1 bis 4, **dadurch gekennzeichnet, dass** am Rahmen (4) mindestens ein Führungselement (9) und mindestens ein Arretierungselement (10) zum Führen und Positionieren der Führungsaufnahme (5) angeordnet sind.

6. Sägelehre nach Anspruch 5, **dadurch gekennzeichnet, dass** in der radial nach außen weisenden Umfangsfläche des Grundkörpers (6) mindestens eine Nut (11) zur Aufnahme jeweils eines am Rahmen (4) gelagerten Führungselements (9) ausgebildet ist.

7. Sägelehre nach Anspruch 6, **dadurch gekennzeichnet, dass** am Rahmen (4) zwei sich im wesentlich einander gegenüberliegend angeordnete Führungselemente (9) gelagert sind.

8. Sägelehre nach Anspruch 6 oder 7, **dadurch gekennzeichnet, dass** das mindestens eine Führungselement (9) als derart in den Rahmen (4) einschraubbare Schraube ausgebildet ist, dass das den Rahmen (4) durchragende freie Ende der Schraube führend in die Nut (11) des Grundkörpers (6) eingreift.

9. Sägelehre nach mindestens einem der Ansprüche 5 bis 8, **dadurch gekennzeichnet, dass** die Führungsaufnahme (5) über ein als Stellschraube ausgebildetes Arretierungselement (10) in der jeweiligen Verschwenkposition innerhalb des Rahmens (4) festlegbar ist.

10. Sägelehre nach mindestens einem der Ansprüche 1 bis 9, **dadurch gekennzeichnet, dass** das Führungselement (2) über mindestens ein Fixierungselement am Sägegut festlegbar ist.

11. Sägelehre nach Anspruch 10, **dadurch gekennzeichnet, dass** das Fixierungselement als am Rahmen und/oder an der Führungsaufnahme (5), insbesondere am Grundkörper (6), ausgebildete Durchgangsbohrung (13) ausgebildet ist, in die ein bis in das Sägegut reichender Haltestift, insbesondere ein Draht (15), einsetzbar ist.

12. Sägelehre nach Anspruch 10, **dadurch gekennzeichnet, dass** das Fixierungselement als am Rahmen (4) und/oder an der Führungsaufnahme (5), insbesondere am Grundkörper (6), ausgebildeter, am Sägegut festlegbarer Fixierungsdorn ausgebildet ist.

13. Sägelehre nach mindestens einem der Ansprüche 1 bis 12, **dadurch gekennzeichnet, dass** der Rahmen (4) auswechselbar an der Handhabe (1) gelagert ist.

14. Sägelehre nach mindestens einem der Ansprüche 1 bis 13, **dadurch gekennzeichnet, dass** die Handhabe (1) als gegenüber dem Sägeblattführungselement (2) abgewinkelter stielförmiger Griff ausgebildet ist.

15. Sägelehre nach Anspruch 14, **dadurch gekennzeichnet, dass** die Handhabe (1) sowohl in horizontaler Richtung als auch in vertikaler Richtung gegenüber dem Sägeblattführungselement (2) abgewinkelt ist.

16. Sägelehre nach mindestens einem der Ansprüche 1 bis 15 , **dadurch gekennzeichnet, dass** die Oberfläche der Handhabe (1), zumindest des distalen Teils der Handhabe (1), gerändelt ausgebildet ist.

## Claims

1. Saw jig for medical purposes, with a handle (1) and with a saw-blade guide element (2) which is mounted on the handle (1) so as to receive and guide a saw blade (3), the saw-blade guide element (2) being composed of a frame (4) mounted on the handle (1), and of a guide seat (5) provided for the saw blade (3) and mounted pivotably in the frame (4), which saw-blade guide element (2) can be fixed on the material to be sawn and, in the position in which it is fixed on the material to be sawn, can be adjusted to different saw cuts, **characterized in that** the frame (4) is configured as a segment of a circle which is open on one side and into which the guide seat (5), comprising a substantially circle-shaped base body (6), can be inserted with a form fit, and a radially outwardly extending web (7) is formed integrally on the base body (6), at least one slit (8) that extends substantially perpendicular to the horizontal extent of the saw-blade guide element (2) being formed in the web (7) for the purpose of receiving the saw blade (3), and the pivot angle of the guide seat (5) within the frame (4) being limited by the web (7) making contact with the end faces (4a) on each side of the opening of the frame (4).

2. Saw jig according to Claim 1, **characterized in that** the slit (8) extends into the base body (6), preferably as far as the centre of the base body (6).

3. Saw jig according to Claim 1 or 2, **characterized in that** the slit (8) for receiving the saw blade (3) is designed wider in the area (8a) of the saw teeth (3a) of the saw blade (3).

4. Saw jig according to at least one of Claims 1 to 3, **characterized in that** the opening of the frame (4) has an angle (α) of less than 180°, preferably 60°.

5. Saw jig according to at least one of Claims 1 to 4, **characterized in that** at least one guide element (9) and at least one locking element (10) are arranged on the frame (4) in order to guide and position the guide seat (5).

6. Saw jig according to Claim 5, **characterized in that** at least one groove (11) is formed in the radially outwardly directed circumferential surface of the base body (6) in order to receive in each case one guide element (9) mounted on the frame (4).

7. Saw jig according to Claim 6, **characterized in that** two guide elements (9) are mounted substantially opposite one another on the frame (4).

8. Saw jig according to Claim 6 or 7, **characterized in that** at least one guide element (9) is designed as a screw that can be screwed into the frame (4) in such a way that the free end of the screw protruding through the frame (4) engages with a guiding function in the groove (11) of the base body (6).

9. Saw jig according to at least one of Claims 5 to 8, **characterized in that** the guide seat (5) can be secured in the respective pivot position inside the frame (4) via a locking element (10) configured as a locking screw.

10. Saw jig according to at least one of Claims 1 to 9, **characterized in that** the guide element (2) can be secured, via at least one fixing element, on the material to be sawn.

11. Saw jig according to Claim 10, **characterized in that** the fixing element is designed as a throughbore (13) which is provided on the frame and/or on the guide seat (5), in particular on the base body (6), and into which it is possible to insert a retention pin, in particular a wire (15), extending into the material to be sawn.

12. Saw jig according to Claim 10, **characterized in that** the fixing element is designed as a fixing spike which is provided on the frame (4) and/or on the guide seat (5), in particular on the base body (6), and which can be secured on the material to be sawn.

13. Saw jig according to at least one of Claims 1 to 12, **characterized in that** the frame (4) is mounted exchangeably on the handle (1).

14. Saw jig according to at least one of Claims 1 to 13, **characterized in that** the handle (1) is designed as a shaft-shaped grip arranged at an angle relative to the saw-blade guide element (2).

15. Saw jig according to Claim 14, **characterized in that** the handle (1) is arranged at an angle relative to the saw-blade guide element (2) both in the horizontal direction and in the vertical direction.

16. Saw jig according to at least one of Claims 1 to 15, **characterized in that** the surface of the handle (1), at least of the distal part of the handle (1), has a knurled finish.

## Revendications

1. Gabarit de sciage pour applications médicales, comportant une manette (1) et un élément guide-lame (2) monté sur la manette (1) et destiné à recevoir et à guider une lame de scie (3), dans lequel l'élément guide-lame (2) constitué par un cadre (4) monté sur la manette (1) et par un logement de guidage (5) monté pivotant dans le cadre (4) et destiné à la lame de scie (3) est susceptible d'être fixé sur l'élément à scier et d'être réglé à différents plans de coupe dans la position fixée sur l'élément à scier,
**caractérisé en ce que**
le cadre (4) est réalisé sous forme de tronçon d'anneau de cercle ouvert sur un côté dans lequel peut être inséré en coopération de formes le logement de guidage (5) comprenant un corps de base (6) réalisé sensiblement en forme circulaire, corps de base (6) sur lequel est conformée une barrette (7) qui s'étend radialement vers l'extérieur, et une fente (8) s'étendant sensiblement perpendiculairement à l'extension horizontale de l'élément guide-lame (2) est ménagée dans la barrette (7) pour recevoir la lame de scie (3), et l'angle de pivotement du logement de guidage (5) à l'intérieur du cadre (4) est limité par l'appui de la barrette (7) contre les deux surfaces frontales (4a) des deux côtés de l'ouverture du cadre (4).

2. Gabarit de sciage selon la revendication 1, **caractérisé en ce que** la fente (8) s'étend jusque dans le corps de base (6), de préférence jusqu'au milieu du corps de base (6).

3. Gabarit selon la revendication 1 ou 2, **caractérisé en ce que** la fente (8) destinée à recevoir la lame de scie (3) est réalisée plus large dans la zone (8a) des dents de scie (3a) de la lame de scie (3).

4. Gabarit de sciage selon l'une des revendications 1 à 3, **caractérisé en ce que** le cadre (4) présente un angle d'ouverture (α) inférieur à 180°, de préférence de 60°.

5. Gabarit de sciage selon l'une des revendications 1 à 4, **caractérisé en ce que** sur le cadre (4) sont agencés au moins un élément de guidage (9) et au moins un élément d'arrêt (10) pour guider et positionner le logement de guidage (5).

6. Gabarit de sciage selon la revendication 5, **caractérisé en ce qu'**il est prévu, dans la surface périphérique du corps de base (6) dirigée radialement vers l'extérieur, au moins une gorge (11) pour recevoir un élément de guidage (9) respectif monté sur le cadre (4).

7. Gabarit de sciage selon la revendication 6, **caractérisé en ce que** deux éléments de guidage (9) agencés sensiblement à l'opposé l'un de l'autre sont montés sur le cadre (4).

8. Gabarit de sciage selon la revendication 6 ou 7, **caractérisé en ce que** ledit au moins un élément de guidage (9) est réalisé sous la forme d'une vis susceptible d'être vissée dans le cadre (4) de telle sorte que l'extrémité libre de la vis traversant le cadre (4) s'engage avec guidage dans la gorge (11) du corps de base (6).

9. Gabarit de sciage selon l'une des revendications 5 à 8, **caractérisé en ce que** le logement de guidage (5) est susceptible d'être immobilisé dans la position de pivotement respective à l'intérieur du cadre (4) via un élément d'arrêt (10) réalisé sous forme de vis de blocage.

10. Gabarit de sciage selon l'une des revendications 1 à 9, **caractérisé en ce que** l'élément de guidage (2) est susceptible d'être immobilisé sur l'élément à scier par au moins un élément de fixation.

11. Gabarit de sciage selon la revendication 10, **caractérisé en ce que** l'élément de fixation est réalisé sous la forme d'un perçage traversant (13) réalisé sur le cadre et/ou sur le logement de guidage (5), en particulier sur le corps de base (6), perçage dans lequel peut être insérée une goupille de maintien pénétrant jusque dans l'élément à scier, en particulier un fil métallique (15).

12. Gabarit de sciage selon la revendication 10, **caractérisé en ce que** l'élément de fixation est réalisé sous la forme d'un mandrin de fixation réalisé sur le cadre (4) et/ou sur le logement de guidage (5), en particulier sur le corps de base (6), et susceptible d'être immobilisé sur l'élément à scier.

13. Gabarit de sciage selon l'une des revendications 1 à 12, **caractérisé en ce que** le cadre (4) est monté de façon interchangeable sur la manette (1).

14. Gabarit de sciage selon l'une des revendications 1 à 13, **caractérisé en ce que** la manette (1) est réalisée sous la forme d'une poignée en forme de tige coudée par rapport à l'élément guide-lame (2).

15. Gabarit de sciage selon la revendication 14, **caractérisé en ce que** la manette (1) est coudée par rapport à l'élément guide-lame (2) aussi bien en direction horizontale qu'en direction verticale.

16. Gabarit de sciage selon l'une des revendications 1 à 15, **caractérisé en ce que** la surface de la manette (1), au moins de la partie distale de la manette (1), est réalisée de façon moletée.
